# EUROPEAN PATENT SPECIFICATION

(11) **EP 2 741 808 B1**
(45) Date of publication and mention of the grant of the patent: **22.03.2017**
(21) Application number: 12822661.0
(22) Date of filing: 10.08.2012
(51) Int. Cl.: A61M 29/00, A61F 2/04, A61F 5/00

(54) **ANCHORING AND DELIVERY SYSTEM FOR A GASTRO-DUODENAL IMPLANT**
VERANKERUNGS- UND FREISETZUNGSSYSTEM FÜR EIN GASTRODUODENALES IMPLANTAT
SYSTÈME D'ANCRAGE ET DE POSE POUR UN IMPLANT GASTRODUODÉNAL

(30) Priority: 10.08.2011 US 201161521773 P
(43) Date of publication of application: 18.06.2014
(60) Divisional application: 17152996.9
(73) Proprietor: National University of Singapore, Singapore 119077 (SG)
(72) Inventor: CHUNG, Sheung Chee, Sydney, Singapore 119077 (SG); SO, Bok Yan , Jimmy, Singapore 119077 (SG); LIM, Jui, Singapore 119077 (SG); LEE, Heow Pueh, Singapore 119077 (SG); LIM, Siak Piang, Singapore 119077 (SG); SHABBIR, Asim, Singapore 119077 (SG); HONG, Tsui Ying, Rachel, Singapore 119077 (SG); LOH, Wee Chuan, Melvin, Singapore 119077 (SG); SIN, Wei Hon, Denis, Singapore 119077 (SG); WONG, Tze Wee, Carolyn, Singapore 119077 (SG)
(74) Representative: dompatent von Kreisler Selting Werner - Partnerschaft von Patent- und Rechtsanwälten mbB
(86) International application number: PCT/SG2012/000285
(87) International publication number: WO 2013/022407

(56) References cited:
- WO-A1-2011/049651
- WO-A1-2012/148727
- WO-A2-2010/074712
- WO-A2-2012/054519
- US-A- 4 315 509
- US-A1- 2003 040 804
- US-A1- 2008 051 823
- US-A1- 2011 046 537

## Description

### Technical Field

The present disclosure relates to bariatric surgery. In particular the present disclosure relates to implantable medical devices for use in bariatric surgery and methods of using the same.

### Background

Obesity is increasing throughout the world at an alarming rate. The World Health Organization (WHO) has projected that in the near future there may be more than two billion overweight adults and more than 700 million obese adults globally. Overweight individuals, especially those who are obese, may experience serious health consequences resulting from the additional weight they carry, such as, cardiovascular disease (e.g., heart disease and stroke), diabetes, musculoskeletal disorders (e.g., osteoarthritis), and some types of cancers (e.g., colon and endometrial).

For those individuals incapable of losing weight using diet and/or exercise, medical devices, methods, and techniques have been attempted for management and treatment of obesity and obesity related conditions. For example, certain types of devices, such as gastric bands and surgical staples, can be used to reduce the size of the stomach, while devices such as implantable gastric stimulants send electrical pulses to the stomach to simulate fullness. Other devices have been developed for implantation within the gastrointestinal tract for modifying absorption of food material by the gastrointestinal walls. For example, endoluminal sleeves block absorption in the duodenum and upper jejunum. Such devices generally include an anchor structure for securing the device within the gastrointestinal tract, and a chute or tube extending away from the anchor structure along portions of the gastrointestinal tract. Food material that is traveling through the chute is at least substantially prevented from coming into direct physical contact with the gastrointestinal walls, thereby facilitating a reduction in absorption of the food by the gastrointestinal walls.

An example of a device that is implantable within the gastrointestinal tract for inhibiting the absorption of food material through the gastrointestinal walls is described in United States Published Patent Application Number 2009/0248171. The gastrointestinal implant device of US 2009/0248171 utilizes a stent-type anchor for securing the device within the gastrointestinal tract relative to the pylorus, and a flexible sleeve that extends from the anchor into the duodenum, which limits the absorption of nutrients by the gastrointestinal walls. However, the anchor of US 2009/0248171 relies upon a set of barbs adapted for penetration into or through gastrointestinal tissue in order to retain the anchor in position. The penetration of an external barb into a gastrointestinal wall can cause irritation or give rise to tissue damage that may result in internal bleeding or infection.

A gastrointestinal implant device having atraumatic surfaces is disclosed in United States Patent No. 5,820,584, which describes a duodenal insert with an elongated tube for reducing intermixing of digestive fluids with partially digested food materials. The duodenal insert of US 5,820,584 is anchored within the pylorus by a pair of rings or cuffs disposed relative to each other in a trans-pyloric manner. While the duodenal insert of US 5,820,584 may have atraumatic surfaces, it can be considered to include various structural, functional, and/or operational limitations, which can hinder the insertion, deployment, reliable retention, and/or removal of the duodenal insert.

Document US4315509 A discloses a gastro-duodenal implant according to the preamble of claim 1.

A need exists for more effective, more reliable, substantially atraumatic gastrointestinal implant devices, methods, and techniques.

### Summary

In accordance with an aspect of the present disclosure, a gastro-duodenal implant includes a first retention structure configured for occupying a portion of the stomach; a second retention structure configured for positioning essentially adjacent to the pyloric sphincter on the duodenal side of the pyloric opening; and a set of resilient coupling members (e.g., at least one resilient coupling member, or a plurality of resilient coupling members) configured for resiliently coupling the first retention structure and the second retention structure, wherein the set of resilient coupling members facilitates resilient biasing of the first retention structure away from the second retention structure.

The set of resilient coupling structures includes a spring configured for displacing portions of the first retention structure relative to the second retention structure, and the set of resilient coupling structures facilitates maintaining the first retention structure generally at a default separation distance from the second retention structure. More particularly, the set of resilient coupling members facilitates mechanical absorption and redistribution of gastrointestinal forces to actively bias the first retention structure away from the second retention structure.

The first retention structure has a spatial extent that significantly exceeds an expected cross sectional area of the pyloric opening, as well as an expected cross-sectional area of the antrum cardiacum. In an embodiment, the first retention structure includes at least two frame members (e.g., linear and/or arcutate frame members) joined together by spring elements to form a default spatial configuration. The first retention structure can include a tiered structure. The first retention structure can include an enclosure having one of a generally ellipsoidal and a generally toroidal shape. For instance, the first retention structure further can include at least two frame members disposed within the enclosure and joined together by spring elements.

In an embodiment, the first retention structure includes a deformable scaffold assembly having a plurality of elements (e.g., hollow or solid elements), the plurality of elements including one of generally ellipsoidal elements and generally toroidal elements. Elements within the plurality of elements of the deformable scaffold structure can be coupled in a side-by-side manner by at least one of non-resilient segments and resilient segments which predictably restrict relative motion between the elements of the deformable scaffold structure.

The deformable scaffold assembly is coupled to the second retention structure by a plurality of coupling members within the set of coupling members. In an embodiment, each element of the deformable scaffold assembly is individually coupled to the second retention structure by a coupling member within the set of coupling members.

With respect to the second retention structure, the second retention structure includes a first ring having a cross-sectional area smaller than the cross-sectional area of the default spatial configuration of the first retention structure and larger than the expected cross-sectional area of a relaxed pyloric opening. The second retention structure can also include a second ring coupled to the first ring by way of a flexible sleeve. In an embodiment, at least one of the first ring and the second ring includes an inflatable cuff. In an embodiment, the second retention structure includes one of a tubular member (e.g., a generally rigid tubular member) and a stent (e.g., a stent scaffold or mesh, such as a metallic scaffold or mesh).

The gastro-duodenal implant can further include at least one chute coupled to the second retention structure and configured for selective fluid communication through the duodenum.

In accordance with another aspect of the present disclosure, a gastro-duodenal implant includes a compressible fluidly distortable first retention structure configured to reside in the stomach; and a second retention structure coupled to the first retention structure, the second retention structure configured for positioning essentially adjacent to the pyloric sphincter on the duodenal side of the pyloric opening. The first retention structure has elasticity sufficient to adaptively deform in response to forces exerted by stomach smooth musculature thereupon, and to resiliently return toward a default spatial configuration. For instance, the first retention structure has a shape that distorts in response to peristaltic forces. Portions of the first retention structure can be compressed and other portions of the first retention structure can expand as a result of fluid displacement within the first retention structure.

The first retention structure can include a set of internal valves to facilitate fluid displacement in response to pressure exerted by stomach musculature upon the first retention structure. Additionally or alternatively, the first retention structure includes a set of radial expansion control elements that extend between interior borders the first retention structure. The set of radial expansion control elements can include internal connectors that are one of rigid and semi-rigid spring-like.

The first retention structure can include a plurality of fluid-filled members coupled together to form a tiered structure. Compression of a fluid filled member corresponding to a given tier of the first retention structure results in fluid transfer to and expansion of portions of one or more other fluid filled members corresponding to one or more other tiers of the first retention structure.

Also described herein is a method of reducing caloric intake and absorption including: inserting a gastro-duodenal implant such that a first retention structure is resident in the stomach and a second retention structure is resident on a duodenal side of the pyloric opening.

### Brief Description of the Drawings

FIG. 1A and FIG. 1B are schematic illustrations depicting aspects of stomach related anatomy and anatomical terminology associated therewith;
FIG. 2A and FIG. 2B are schematic illustrations of a gastro-duodenal implant according to an embodiment of the disclosure;
FIG. 2C is a schematic illustration of a first retention structure according to an embodiment of the disclosure, and FIG. 2D is an image of a corresponding prototype first retention structure;
FIG. 2E is a schematic of a prototype tiered first retention structure based upon the prototype first retention structure shown in FIG. 2D;
FIG. 2F and FIG. 2G are schematic illustrations of representative types of second retention structures according to embodiments of the disclosure;
FIG. 2H is a schematic illustration of a gastro-duodenal implant according to another embodiment of the disclosure and deployed within a subject;
FIG. 2I is a schematic illustration showing a set of band ligature elements that can form portions of a gastro-duodenal implant according to an embodiment of the disclosure;
FIG. 3A is a schematic planar view of a first retention structure disposed within a generally ellipsoidal enclosure, casing, or shell according to an embodiment of the disclosure;
FIG. 3B is a schematic illustration of portions of a representative gastro-duodenal implant deployed within a subject and having a first retention structure disposed within the generally ellipsoidal enclosure of FIG. 3A;
FIG. 3C is a schematic planar view of a first retention structure disposed within a generally toroidal enclosure, casing, or shell according to an embodiment of the disclosure;
FIG. 4A is a schematic illustration of a gastro-duodenal implant according to another embodiment of the disclosure;
FIG. 4B is a schematic illustration of a gastro-duodenal implant according to a further embodiment of the disclosure;
FIG. 5A is a schematic illustration of a gastro-duodenal implant according to another embodiment of the disclosure and deployed within a subject;
FIG. 5B is a schematic top planar view of a fluidly distortable or deformable first retention structure of the gastro-duodenal implant of FIG. 5A;
FIG. 5C is a schematic illustration showing portions of the gastro-duodenal implant of FIG. 5A under a relaxed antrum condition;
FIG. 5D is a schematic illustration showing portions of the gastro-duodenal implant of FIG. 5A under a contracted antrum condition;
FIG. 5E is a schematic-illustration showing an internal expansion regulator or connector within the fluidly distortable first retention structure of FIG. 5A under a relaxed antrum condition;
FIG. 5F is a schematic illustration showing internal expansion regulators or connectors within the fluidly distortable first retention structure of FIG. 5A under a contracted antrum condition;
FIG. 6A and FIG. 6B are schematic illustrations showing portions of gastro-duodenal implant according to further embodiments of the disclosure, which include a plurality of fluid filled members that are fluidly coupled together, and which form a tiered type of fluidly deformable first retention structure;
FIG. 7A is a schematic illustration of an implantation and deployment capsule configured for carrying a gastro-duodenal implant in a concentric manner around a portion of an endoscope according to an embodiment of the disclosure;
FIG. 7B is a schematic illustration of an implantation and deployment capsule configured for carrying a gastro-duodenal implant in an offset manner relative to an endoscope according to an embodiment of the disclosure;
FIG. 7C is a schematic illustration of an implantation and deployment capsule in an "in-line" configuration with respect to an endoscope;
FIG. 7D is a schematic illustration of an implantation and deployment capsule in an "offset" configuration with respect to an endoscope;
FIG. 8 is a schematic illustration showing a first retention structure disposed within a portion of an implantation and deployment capsule according to an embodiment of the disclosure; and
FIG. 9 is a schematic illustration of an implantation and deployment capsule that includes a serpentine portion surrounding a segment of an endoscope according to an embodiment of the disclosure.

### Detailed Description

In the present disclosure, depiction of a given element or consideration or use of a particular element number in a particular FIG. or a reference thereto in corresponding descriptive material can encompass the same, an equivalent, or an analogous element or element number identified in another FIG. or descriptive material associated therewith. Additionally, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented herein.

As used herein, the term "set" corresponds to or is defined as a non-empty finite organization of elements that mathematically exhibits a cardinality of at least 1 (i.e., a set as defined herein can correspond to a singlet or single element set, or a multiple element set), in accordance with known mathematical definitions (for instance, in a manner corresponding to that described in An Introduction to Mathematical Reasoning: Numbers, Sets, and Functions, "Chapter 11: Properties of Finite Sets" (e.g., as indicated on p. 140), by Peter J. Eccles, Cambridge University Press (1998)). In general, an element of a set can include or be a system, an apparatus, a device, a structure, a structural feature, an object, a process, a physical parameter, or a value depending upon the type of set under consideration.

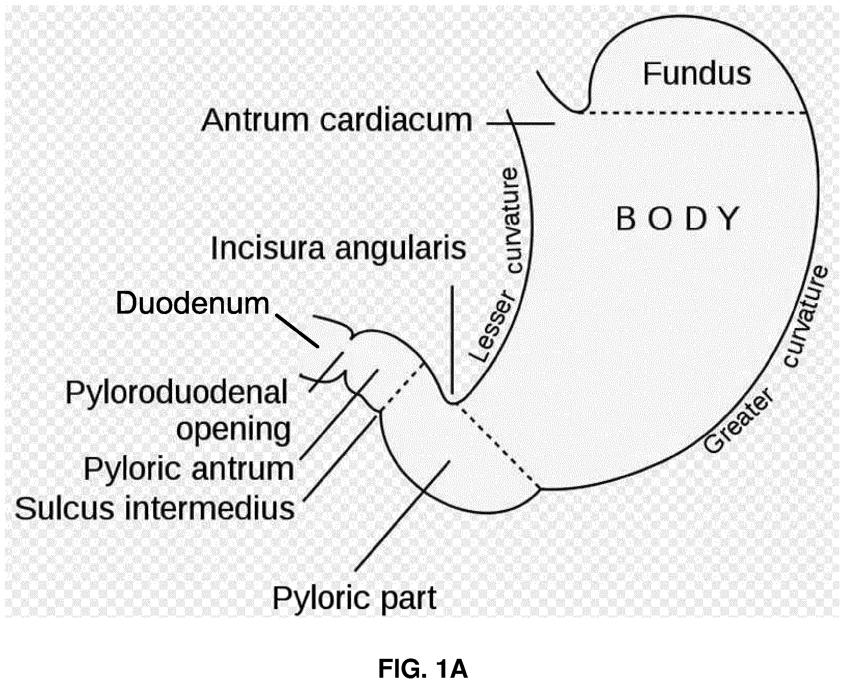

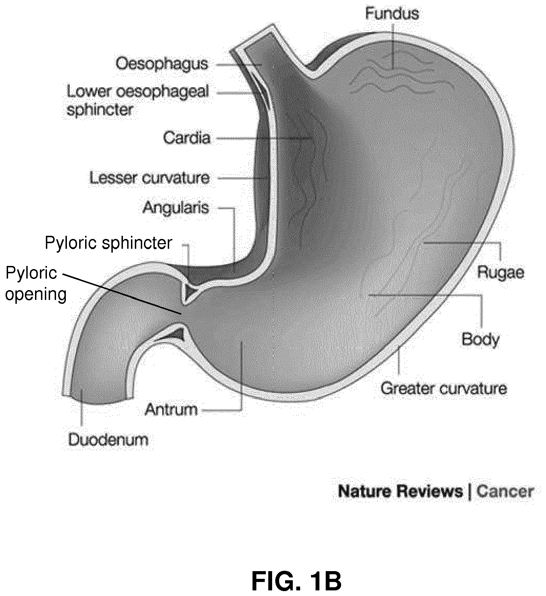

FIG. 1A and FIG. 1B are schematic illustrations showing particular aspects of stomach related anatomy and corresponding anatomical terminology referenced in the description below.

Particular aspects of the present disclosure relate to a substantially atraumatic gastro-duodenal implant that can be reliably maintained at intended positions along portions of the gastrointestinal tract by way of a (1) first retention structure that includes structural elements residing within portions of the stomach at a significant distance away from an antral, superior, or proximal side of the pyloric opening, and having a spatial extent that minimizes a likelihood of accommodation by the pyloric sphincter; (2) a second retention structure disposed on a duodenal, inferior, or distal side of the first retention structure, across the pyloric opening; and (3) a number of resiliently deformable (flexible) elements or structural segments that facilitate mechanical absorption of gastrointestinal forces, displacement of certain portions of the first retention structure relative to other portions of the first retention structure, displacement of portions of the first retention structure relative to the second retention structure, and/or resilient biasing of the first retention structure away from the pyloric opening.

Gastro-duodenal implants in accordance with aspects of the present disclosure can further include a chute extending away from the second retention structure into a portion of the duodenum or beyond for modifying intestinal absorption of substances.

Embodiments of the present disclosure are directed to a substantially or essentially atraumatic gastro-duodenal implant that can be reliably retained, maintained, or anchored along or at intended portions of the gastrointestinal tract relative to the pyloric opening and/or the pyloric sphincter. Various embodiments of a gastro-duodenal implant in accordance with the present disclosure include the following:
(1) a stomach resident first retention structure that has a set of structural features, segments, or elements configured to reside a significant distance away from an antral, superior, or proximal side of the pyloroduodenal or pyloric opening, and which has a spatial extent or cross sectional area that significantly exceeds a maximum or expected maximum cross sectional area of each of the pyloric opening and the antrum cardiacum in order to reduce or minimize the likelihood that substantial or significant portions of first retention structure can be accommodated by the pyloric sphincter or the esophagus, respectively;
(2) a second retention structure that includes one or more elements configured to reside on a duodenal, inferior, or distal side of the pyloric opening, which has a spatial extent or cross sectional area that is significantly smaller than the cross sectional area of the first retention structure yet larger than the cross sectional area of the relaxed pyloric opening, and which can be accommodated by a portion of the gastrointestinal tract along the duodenal side of the pyloric opening without tissue damage;
(3) a number of resilient, deformable, or elastic elements or structural segments configured to maintain the first retention structure away from the second retention structure at a default or average separation distance in the absence or substantial absence of gastrointestinal forces (e.g., compressive and/or displacement forces resulting from smooth muscle contractions, such as associated with peristalsis) exerted upon the gastro-duodenal implant, and further configured to resiliently respond or adapt to forces exerted upon the gastro-duodenal implant in a manner that facilitates displacement of portions of the first retention structure relative to the second retention structure; and in several embodiments,
(4) a flexible sleeve, tube, or chute extending away from the second retention structure into a portion of the duodenum for modifying, limiting, and/or preventing intestinal absorption of substances such as chyme or ingested food matter.

A set of resilient or elastically deformable elements or structural segments, in accordance with the present disclosure, can include a number of spring type or spring based elements or structural segments, and/or a number of fluidly deformable (e.g., having selectively expandable or inflatable and contractable or deflatable segments or sections) elements or structural segments. As further detailed below, particular resilient or elastically deformable elements or structural segments can be carried by or between portions of coupling members that extend between the first and second retention structures, and certain resilient or elastically deformable elements or structural segments can be carried by or between portions of the first retention structure itself.

Resilient or elastically deformable elements, in accordance with embodiments of the present disclosure, are configured to respond to gastrointestinal forces by at least partially absorbing and (re)distributing gastrointestinal forces, facilitating or enabling displacement or translation of certain portions of the first retention structure relative to other portions of the first retention structure, and/or facilitating or enabling displacement or translation of portions of the first retention structure relative to (e.g., towards and/or away from) the second retention structure. As gastrointestinal forces decrease or become minimal or substantially absent, the resilient or elastically deformable elements position or maintain the first retention structure toward or at the default separation distance from the second retention structure, such that the first retention structure is displaced toward or generally maintained or disposed at a default or resting region or position within the stomach away from the antral side of the pyloric opening.

A default separation distance between the first retention structure and the second retention structure can be approximately 3.5 - 18.5 cm (e.g., about 5.0 - 15.0 cm, or about 10.0 - 15.0 cm), depending upon embodiment details and/or the direction and magnitude of gastrointestinal forces under consideration. Correspondingly, the first retention structure's default or resting position within the stomach can be approximately 2.5 - 17.5 cm (e.g., about 4.0 - 14.0 cm, or about 9.0 - 14.0 cm) away from the antral side of the pyloric opening.

As indicated above, the first retention structure has a spatial extent or cross sectional area that is significantly greater than the spatial extent or cross sectional area of the second retention structure in order to facilitate reliable gastro-duodenal implant retention within a subject. Additionally, the first retention structure can have a spatial extent or cross sectional area that is intended to increase or maximize the likelihood that peripheral portions of the first retention structure come into contact with the stomach lining on a regular or generally continuous basis, or following a subject's ingestion of food and/or during food churning motions provided by the stomach's smooth musculature. As a result, the first retention structure can contribute to or provide a satiety effect (e.g., as a result of portions of the first retention structure contacting visceral sensory nerves in the stomach lining, which generate afferent satiety signals that mediate the subject's perception of having a full stomach) on a sustained basis, or following the intake of a small, reasonably small, or relatively limited quantity or volume of food. A gastro-duodenal implant according to an embodiment of the disclosure can therefore simultaneously serve as a satiety inducement, triggering, prolongation, or maintenance device, as well as an intestinal absorption modification device. That is, a gastro-duodenal implant according to an embodiment of the disclosure can simultaneously provide dual-mode therapeutic functionality corresponding to satiety enhancement and intestinal absorption modification.

Embodiments of the present disclosure can be categorized as atraumatic or essentially atraumatic in that they are intended to avoid gastrointestinal tissue penetration, for instance, by way of structural elements or features that are formed using soft, smooth, flexible, atraumatic materials such as particular types of polymer compositions indicated below. Various embodiments of the present disclosure exclude structural features or elements that are intended to penetrate into or through the gastrointestinal lining, such as barbs, or tethering elements that are configured to structurally retain particular portions of a gastro-duodenal implant in a given position or orientation by way of a tether that extends through the gastrointestinal lining to an anatomical structure external to the gastrointestinal lining (e.g., an outer portion of the stomach, or a visceral structure).

Embodiments of the present disclosure can form portions of a gastro-duodenal anchoring system that can dynamically and reliably maintain an intestinal absorption modification sleeve or chute in an intended or desired position in the presence of contractive gastrointestinal forces such as peristalsis, and which can provide or enhance a satiety effect. A gastro-duodenal anchoring system in accordance with an embodiment of the disclosure can be used to treat aspects of obesity, eating disorders, diabetes, or other conditions. Particular embodiments of the present disclosure are additionally directed to aspects of a delivery system configured for implantation of the gastro-duodenal anchoring system within appropriate portions of the gastrointestinal tract of a subject (e.g., a human or other mammal).

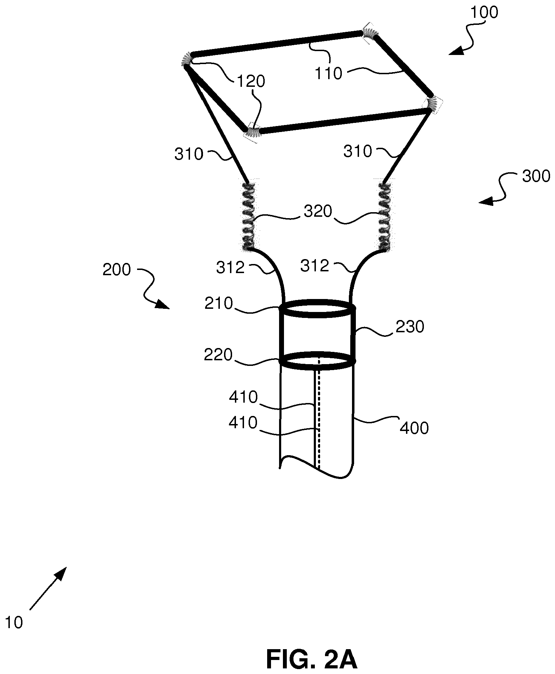

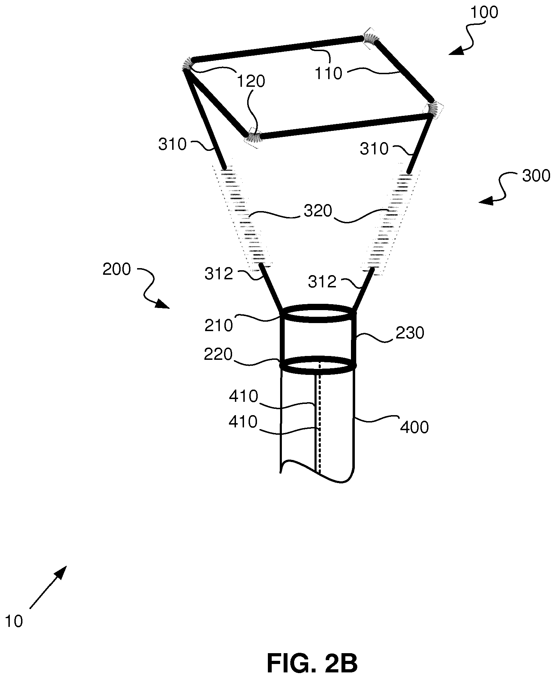

FIG. 2A and FIG. 2B are schematic illustration of a gastro-duodenal implant 10 according to an embodiment of the disclosure. In an embodiment, the gastro-duodenal implant 10 includes a first retention structure 100 configured for occupying a portion of the stomach's volume; a second retention structure 200 configured for positioning adjacent or essentially adjacent to the pyloric sphincter on the duodenal side of the pyloric opening; and a set of coupling members 300 configured for resiliently or elastically coupling the first retention structure 100 and the second retention structure 200, and maintaining the first retention structure 100 at or generally at or returning the first retention structure 100 to or generally to a predetermined default or resting position away from the second retention structure 200 (e.g., in the absence or substantial absence of gastrointestinal forces exerted upon the gastro-duodenal implant 10).

This gastro-duodenal implant 10 embodiment further includes a chute 400 configured for positioning along portions of the intestine (e.g., the duodenum), which can modify, limit, and/or prevent the intestinal absorption of substances. In some embodiments, the chute 400 carries or includes one or more channels or passages 410 configured for selective fluid communication (e.g., air and/or liquid transfer) with portions of the second retention structure 200, and which facilitate deployment or extension of the chute 400 along portions of the intestinal tract.

The first retention structure 100 includes a plurality of frame members 110 that are resiliently coupled by way of spring elements or springs 120. The springs 120 dispose the frame members 110 in a default spatial configuration in the absence of forces applied to or exerted upon the first retention structure 100. Depending upon embodiment details and/or the nature of the first retention structure's default spatial configuration, the frame members 100 can be separated or spaced apart from each other by approximately 5.0 - 10.0 cm (e.g., about 7.0 - 8.0 cm). In an embodiment in which the first retention structure 100 exhibits a generally square default spatial configuration, a frame member separation distance of approximately 7.0 - 8.0 cm can facilitate reliable retention of the gastro-duodenal implant within the subject as well as inducement, enhancement, prolongation, or maintenance of subject satiety.

Each spring 120 can include a first terminal that is coupled to a first portion, segment, or end of a given frame member 110, and a second terminal that is coupled to a second portion, segment, or end of another frame member 110. In various embodiments, the springs 120 are bonded and/or welded (e.g., laser or ultrasonically welded) to the frame members 110. The springs 120 can include linear or essentially linear springs, such as helical-type and/or flat springs. In multiple embodiments, the frame members 110 can have an outer diameter of approximately 0.5 - 1.0 cm; the springs 120 can have an outer diameter of approximately 0.5 - 1.0 cm; and the springs 120 can include polymer and/or metal materials that can have a spring constant between approximately 10 - 75 N/m (e.g., approximately 20 - 60 N/m, or about 50 N/m).

The springs 120 facilitate or enable a certain amount of movement or displacement of frame members 110 relative to each other in response to forces exerted upon the first retention structure 100. Additionally, the springs 120 resiliently urge or bias the frame members 110 toward or to the first retention structure's default spatial configuration when such forces decrease or are absent or substantially absent. As a result, the shape of the first retention structure 100 can resiliently distort or deform in response to forces exerted by the stomach's smooth musculature (e.g., stomach contraction forces) upon portions of the first retention structure 100.

In various embodiments, the second retention structure 200 includes a first ring or opening 210 that is intended to reside closest to the pyloric opening; a second ring or opening 220 that is intended to reside distal to the pyloric opening relative to the first opening 210; and a body segment, section, or structure 230 that extends between the first and second openings 210, 220. The chute 400 can extend from the first opening 210 to and past the second opening 220 into portions of the intestinal tract. The second retention structure 200 can exhibit multiple types of structural configurations depending upon embodiment details, as further described below.

The coupling members 300 can include a number of linkage elements 310, 312 that are themselves coupled by way of spring elements or coupling member springs 320. For instance, a given coupling member 300 can include a first linkage element 310 that is coupled to a spring 120 of the first retention structure 100, and which is further coupled to a coupling member spring 320 carried by the coupling member 300. This coupling member 300 additionally includes a second linkage element 312 that is coupled to the coupling member spring 320, and which is further coupled to one or more portions of the second retention structure 200. In various embodiments, the aforementioned couplings between the first retention structure 100 and the first linkage element 310; the first linkage element 310 and the coupling member spring 320; the coupling member spring 320 and the second linkage element 312; and the second linkage element 312 and the second retention structure 200 can be made by way of bonds or welds (e.g., laser or ultrasonic welds).

The linkage elements 310, 312 and/or springs 320 can be of desired or different lengths in order to accommodate expected stomach curvature and facilitate positioning of the first retention structure 100 at a default position or region within the stomach. Furthermore, the linkage elements 310, 312 can be semi-rigid or at least slightly / somewhat resilient (e.g., a linkage element 310, 312 can include a flat spring). The coupling member springs 320, possibly in association with the linkage elements 310, 312, resiliently absorb longitudinal or lengthwise (e.g., with reference to a direction of food transit from the stomach to the intestines) forces (such as peristaltic or emetic forces) exerted by stomach smooth musculature which alter the separation distance between the first and second retention structures 100, 200. As such forces subside, the coupling member's springs 320 in association with the linkage elements 310, 312 resiliently reestablish the default separation distance between the first and second retention structures 100, 200, thereby returning the first retention structure 100 toward or to its default position within the stomach.

In multiple embodiments, one or more coupling members 300 has an overall length of approximately 5.0 - 17.0 cm (e.g., about 10.0 - 15.0 cm), and a maximum or average thickness or diameter of approximately 1.5 - 8.5 mm (e.g., about 2.5 - 7.5 mm, or about 5.0 mm). In some embodiments, a linkage element 310, 312 have a length of approximately 2.0 - 8.0 cm (e.g., about 4.0 - 6.0 cm, or about 5.0 cm); and a coupling member spring 320 can have a length of approximately 4.0 - 12.0 cm (e.g., about 6.0 - 10.0 cm, or about 8.0 cm). The spring constant of a coupling member spring 320 can fall within a range such as that provided above for the first retention structure 300.

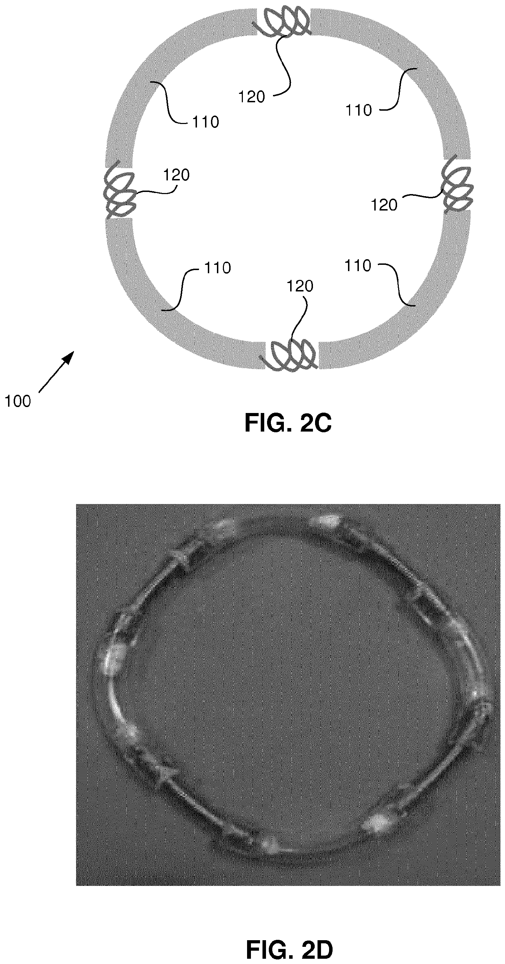

While the embodiment of the first retention structure 100 shown in FIG. 2A includes four frame members 110 coupled to four springs 120 in a manner that provides the first retention structure 100 with a generally rectangular or square default spatial configuration, other embodiments can include one or more frame members 110 with or without the inclusion of springs 120 and/or exhibit a different type of default spatial configuration. For instance, FIG. 2C is a schematic illustration of a first retention structure 100 according to an embodiment of the disclosure, and FIG. 2D is an image of a corresponding prototype first retention structure 100. As shown in FIGs. 2C and 2D, this embodiment of the first retention structure 100 exhibits a generally or substantially elliptical or circular shape, which is formed by arcuate frame members 110 that are coupled by spring elements or springs 120.

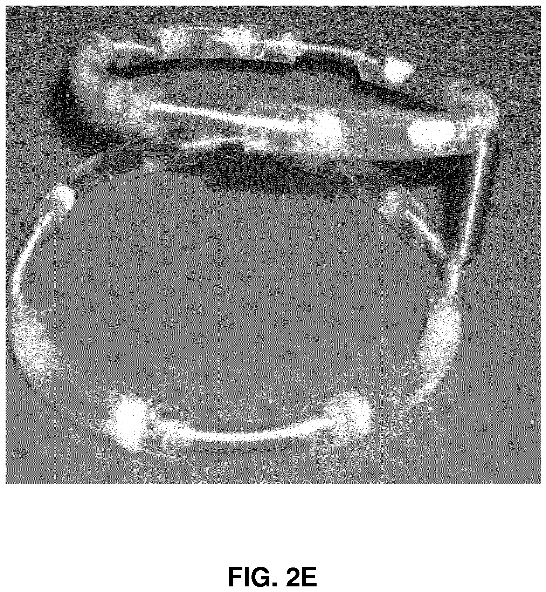

In some embodiments, the first retention structure 100 exhibits a tiered or multi-level structure, which can provide the first retention structure 100 with the ability to accommodate (e.g., resiliently absorb and redistribute) more intense forces (such as peristaltic or emetic forces) exerted by the stomach's smooth musculature upon the first retention structure 100. FIG. 2E is a schematic of a prototype tiered first retention structure 102 based upon the prototype first retention structure 100 shown in FIG. 2D.

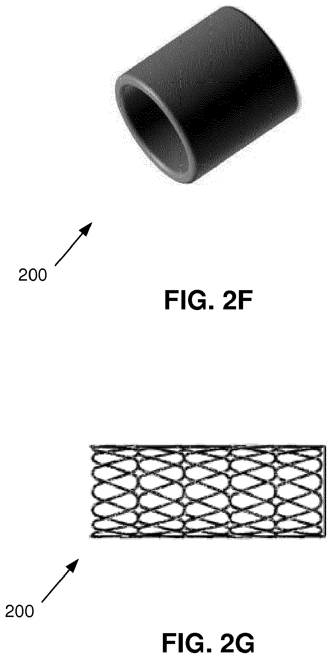

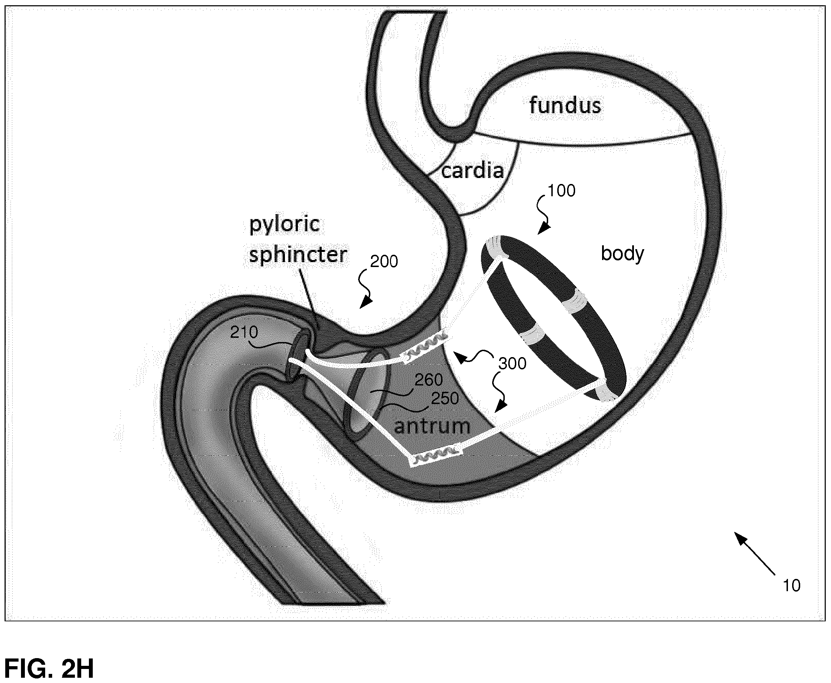

As indicated above, embodiments of the gastro-duodenal implant can encompass various types of second retention structures. FIGs. 2F and 2G are schematic illustrations of representative types of second retention structures 200 according to embodiments of the disclosure. An embodiment such as that shown in FIG. 2F includes a rigid or generally rigid tubular member. In a particular embodiment such as that shown in FIG. 2G, the second retention structure 200 includes a duodenal stent (e.g., a stent scaffold or mesh, such as a tubular metallic scaffold or mesh). FIG. 2H is a schematic illustration of a gastro-duodenal implant 10 according to another embodiment of the disclosure and deployed within a subject, which includes a transpyloric second retention structure 200 having a duodenal side cuff 210 that is coupled to an antral side cuff 250 by way of a flexible sleeve 260. The duodenal side cuff 210 and/or the antral side cuff 250 can be fluid inflatable.

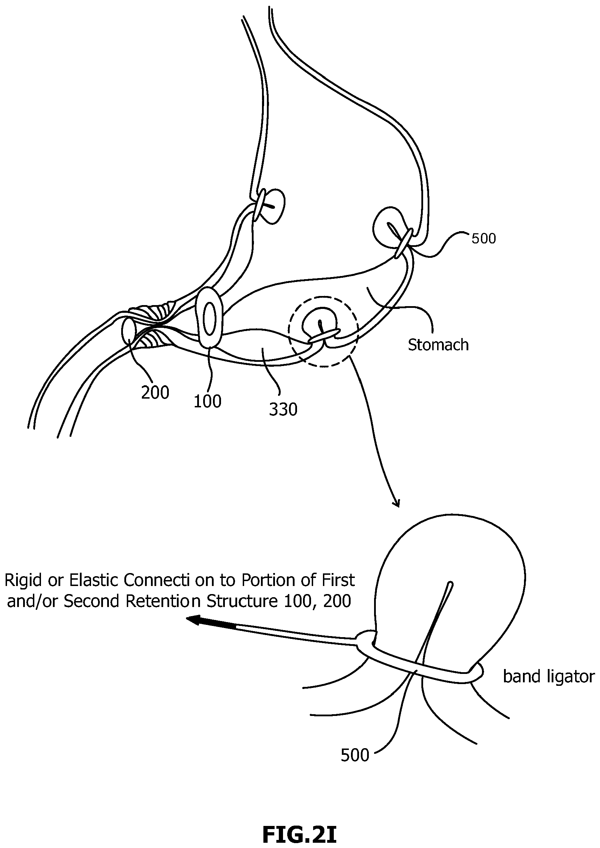

Certain embodiments of the present disclosure can additionally include one or more other types of devices configured for facilitating or enabling reliable gastro-duodenal implant anchoring or spatial positioning within a subject. For instance, FIG. 2I is a schematic illustration showing a set of band ligator elements 500 for non-abrasively anchoring or attaching the gastro-duodenal implant within the stomach and, which can form portions of a gastro-duodenal implant according to an embodiment of the disclosure. In such an embodiment, one or more band ligator elements 500 can be coupled or attached to portions of the first and/or second retention structures 100, 200 via coupling members 330. In particular embodiments, couplings 330 between a given band ligator element 500 and a portion of the first and/or second retention structures 100, 200 can include resilient or elastic elements such as springs.

Portions of a gastro-duodenal implant in accordance with the present disclosure, which can come into contact with a subject's gastrointestinal lining, include or are composed of soft, pliable, materials that are expected to be atraumatic or essentially atraumatic on a long term, lasting, or essentially permanent basis, such as hard or soft plastics, polymers, or copolymers (e.g., one or more of PTFE, polycarbonate, polypropylene, silicone, Chronoflex, and Chronosil). Portions of a gastro-duodenal implant such as springs 120, 320 that can include metallic materials (e.g., stainless steel or Nitinol) can be coated or encapsulated with such atraumatic materials.

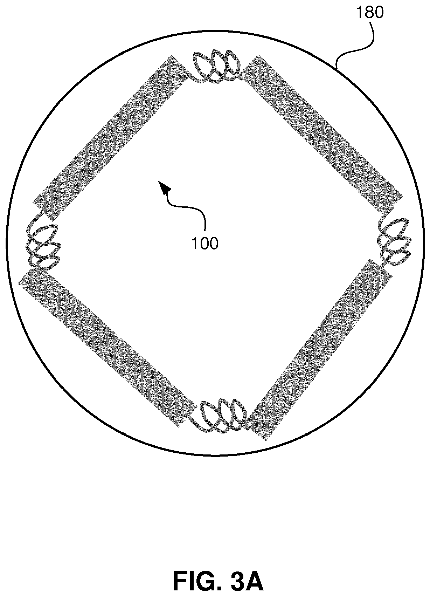

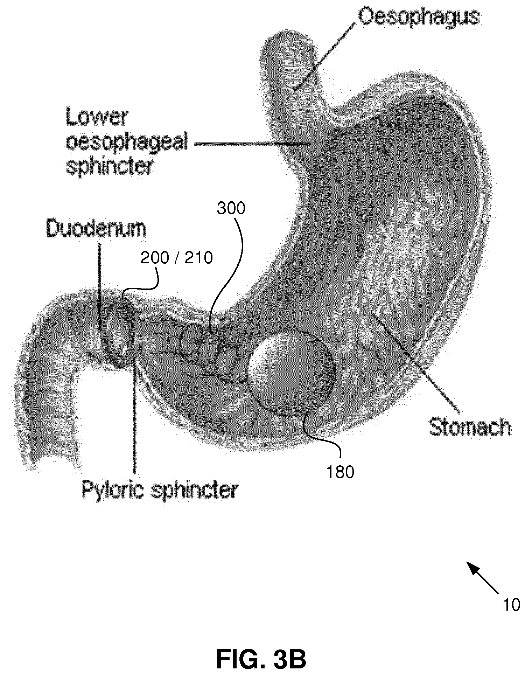

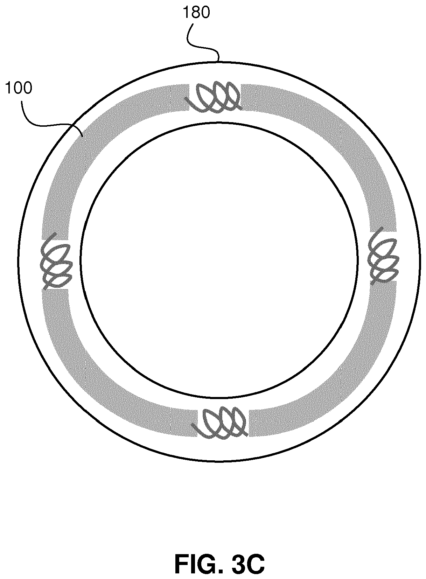

In addition to the foregoing, in certain embodiments, the first retention structure 100 can be carried within portions of an atraumatic structure in order to further enhance the likelihood that a gastro-duodenal implant is atraumatic. For instance, FIG. 3A is a schematic planar view of a first retention structure disposed within a generally ellipsoidal enclosure, casing, or shell (e.g., a balloon type device) 180 according to an embodiment of the disclosure, and FIG. 3B is a schematic illustration of portions of a representative gastro-duodenal implant deployed within a subject and having a first retention structure disposed within the generally ellipsoidal enclosure 180 of FIG. 3A. As an alternative to the foregoing, an atraumatic structure configured to carry the first retention structure 100 can have a generally toroidal shape. FIG. 3C is a schematic planar view of a first retention structure disposed within a toroidal enclosure, casing, or shell 180 according to an embodiment of the disclosure.

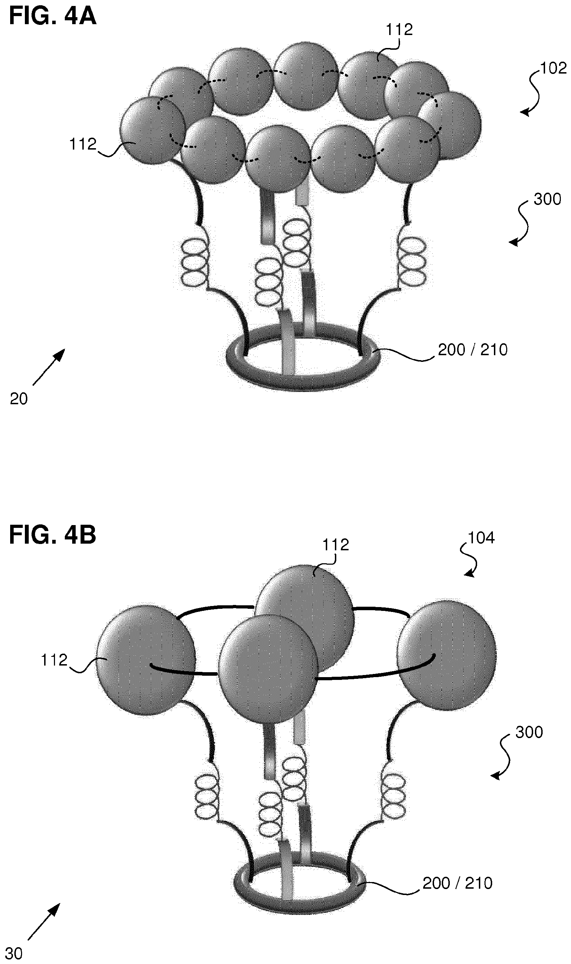

FIG. 4A is a schematic illustration of a gastro-duodenal implant 20 according to another embodiment of the disclosure. In an embodiment, the gastro-duodenal implant 20 includes a first retention structure 102 having a plurality of closely spaced (e.g., nearly touching or touching) generally ellipsoidal, spherical, and/or toroidal elements 112 that are coupled to one another to form a flexible or deformable platform or scaffold assembly. In multiple embodiments, the ellipsoidal elements 112 are coupled in a side-by-side manner, such that the first retention structure 102 exhibits a generally toroidal or ring shaped default spatial configuration. One or more ellipsoidal elements 112 can be solid or hollow, and can have an outer dimension or diameter of approximately 1.0 - 2.0 cm.

In response to forces exerted upon portions of the first retention structure 102 by stomach smooth musculature, the couplings between ellipsoidal elements 112 facilitate or enable a certain amount of motion or displacement of the ellipsoidal elements 112 relative to each other. The couplings between ellipsoidal elements 112 can include non-resilient segments or elements (e.g., fixed length linkages) and/or resilient segments or elements (e.g., elastic or spring type segments or elements), which limit or predictably restrict relative motion between the ellipsoidal elements 112. In several embodiments, resilient couplings between the ellipsoidal elements 112 urge, bias, or return the ellipsoidal elements 112 toward or to the default spatial configuration in the absence or substantial absence of forces associated with smooth musculature contraction.

One or more coupling members 300 are interposed between portions of the first retention structure 102 and the second retention structure 200 to facilitate or enable biasing or maintenance of the platform assembly toward or at a default separation distance from the first end 210 of the second retention structure 200 in the absence or substantial absence of forces applied by stomach smooth musculature, and resilient displacement of portions of the first retention structure 102 relative to the second retention structure 200 in response to such forces.

FIG. 4B is a schematic illustration of a gastro-duodenal implant 30 according to a further embodiment of the disclosure. In an embodiment, the gastro-duodenal implant 30 includes a first retention structure 104 having a plurality of generally ellipsoidal, spherical, or toroidal elements 112 that are separated by at least a predetermined minimum default distance, such as a predetermined fraction or percentage (e.g., 25% - 100%) of an ellipsoidal element's outer diameter. The ellipsoidal elements 112 are coupled by way of linkages that can include one or more flexible, elastic, or resilient segments or elements (e.g., spring type elements), such that the gastro-duodenal implant 30 can adaptively deform in response to forces exerted by stomach smooth musculature thereupon, and exist in or resiliently return toward or to a default spatial configuration and a default stomach position in a manner analogous to that described above when such forces subside or are substantially absent.

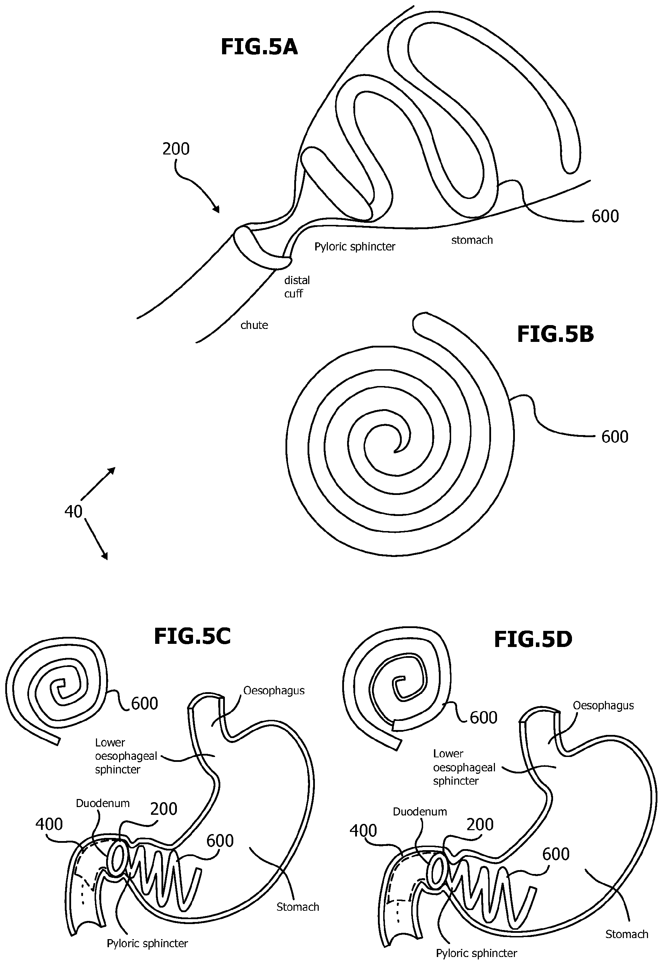

FIG. 5A is a schematic illustration of a gastro-duodenal implant 40 according to another embodiment of the disclosure, deployed within a subject. In an embodiment, the gastro-duodenal implant 40 includes a resiliently compressible / relaxable fluidly distortable or deformable (e.g., expandable / collapsible) first retention structure 600 that is coupled to the second retention structure 200, and which extends away from the second retention structure 200 into portions of the stomach (e.g., proximate to or beyond the incisura angularis). FIG. 5B is a schematic top planar view of the fluidly distortable first retention structure 600 of FIG. 5A, which in an embodiment can exhibit a spiral configuration.

The fluidly distortable first retention structure 600 can include one or more segments or sections containing air or a liquid and can exhibit viscoelastic properties, having elasticity sufficient to adaptively deform in response to forces exerted by stomach smooth musculature thereupon, and exist in or resiliently return toward or to a default spatial configuration. The fluidly distortable first retention structure also has viscous properties, specifically as peristaltic forces contract the upper part of the stomach and food enters the stomach pressure exerted on the upper portion of the first retention structure distorts the shape of the first retention structure, enlarging the lower portion and minimizing the upper portion. The viscous nature of the first retention structure resists change of shape and may thereby induce a feeling of fullness.

In the absence of forces exerted by stomach smooth musculature upon the fluidly distortable first retention structure 600, the fluidly distortable first retention structure 600 maintains a default spatial configuration. In response to forces exerted by stomach smooth musculature, one or more portions of the fluidly distortable first retention structure 600 can be compressed, and other portions of the fluidly distortable first retention structure 600 can expand as a result of fluid displacement within the fluidly distortable first retention structure 600.

FIG. 5C is a schematic illustration showing portions of the gastro-duodenal implant 40 of FIG. 5A under a relaxed antrum condition, and FIG. 5D is a schematic illustration showing portions of the gastro-duodenal implant 40 of FIG. 5A under a contracted antrum condition. Antral contraction causes compression of those portions of the fluidly distortable first retention structure 600 that are directly exposed to such contraction. This compression results in displacement of fluid within the fluid distortable first retention structure 600 away from the antral region toward those portions of the fluid distortable first retention structure 600 that reside within the stomach superior to the antrum (e.g., toward or to the body of the stomach), and expansion (e.g., radial expansion) of such portions of the fluidly distortable first retention structure 600 superior to the antrum in a manner indicated in FIG. 5D. Following cessation of the antral contraction, the shape of the fluid distortable first retention structure 600 returns to a form indicated in FIG. 5C.

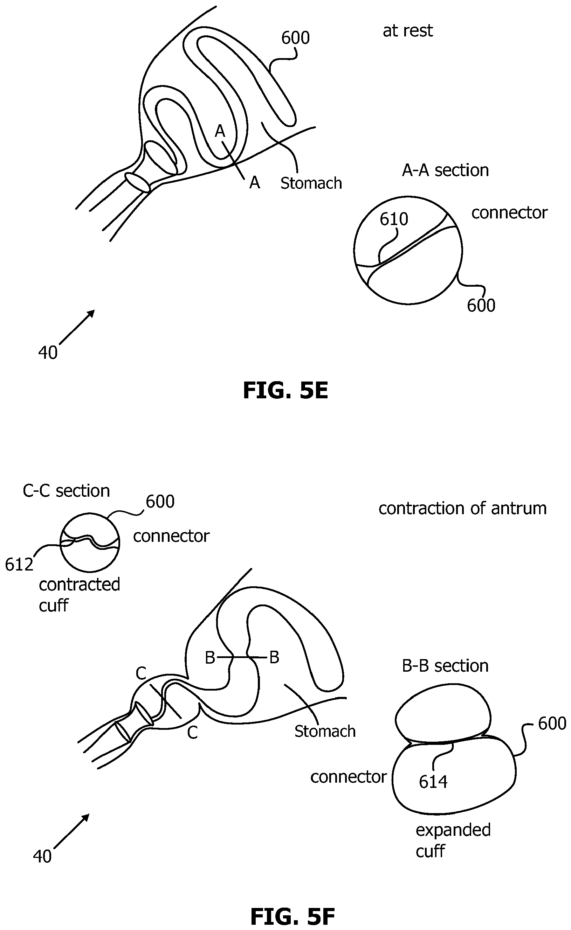

Particular portions of the fluidly distortable first retention structure 600 can include a set of internal valves to facilitate fluid displacement in response to particular levels of pressure exerted by stomach musculature upon the fluidly distortable first retention structure. Additionally or alternatively, the fluid distortable first retention structure 600 can include a number of radial expansion control elements such as rigid connectors that extend between the interior borders or walls of the fluid distortable first retention structure 600. FIG. 5E is a schematic illustration showing an internal connector 610 within the fluidly distortable first retention structure of FIG. 5A under a relaxed antrum condition. FIG. 5F is a schematic illustration showing internal connectors 612, 614 within the fluidly distortable first retention structure of FIG. 5A under a contracted antrum condition. The internal connectors 612, 614 can be rigid to semi-rigid spring-like and work to maintain the structure of the fluidly distortable first retention structure 600.

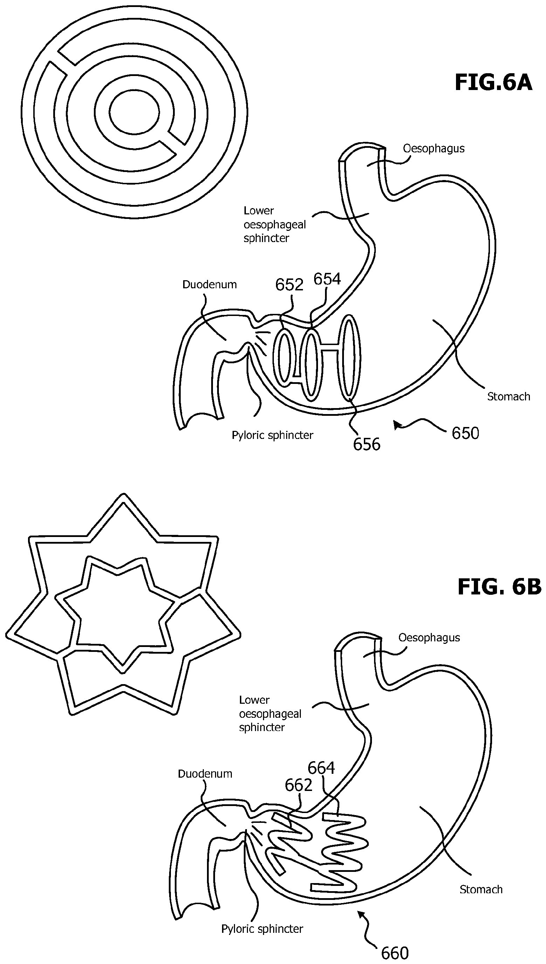

FIGs. 6A / 6B are schematic illustrations showing portions of a gastro-duodenal implant according to further embodiments of the disclosure, which include a plurality of fluid filled members 652, 654, 656 / 662, 664 that are fluidly coupled together in a manner that forms a tiered type of fluidly deformable first retention structure 650 / 660. Compression of a fluid filled member 652, 654, 656 / 662, 664 corresponding to a given tier can result in fluid transfer to and expansion of portions of one or more other fluid filled members 652, 654, 656 / 662, 664 corresponding to one or more other tiers. For instance, compression of a fluid filled member 652, 662 closest to the antral portion of the stomach can result in fluid transfer to and expansion of one or more filled members 654, 656, 664 that reside superior to the fluid filled under compression, in a manner analogous to that described above in relation to FIGs. 5A - 5F.

In accordance with the present disclosure, the gastro-duodenal implant can be implanted by inserting the gastro-duodenal implant such that a first retention structure is resident in the stomach and a second retention structure is resident on a duodenal side of the pyloric opening. In embodiments, the first retention structure and the second retention structure can be connected by coupling elements as depicted in FIGs. 2A, B, H, I, 3B, 4A or 4B. In some embodiments, the first retention structure and the second retention structure can be connected to each other directly as depicted in FIGs. 5A-F. The method of implanting the gastro-duodenal implant can include the use of a device for implantation and deployment.

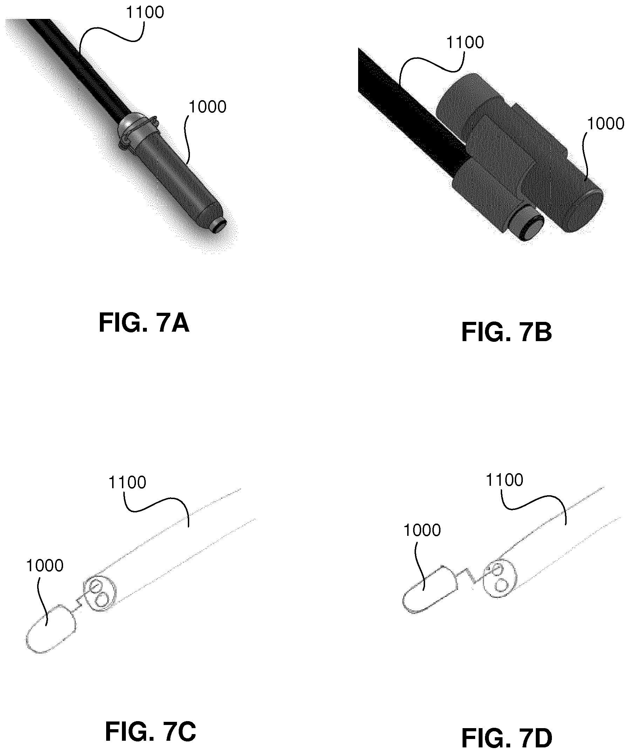

Various embodiments of gastro-duodenal implants in accordance with the present disclosure are configured for implantation and deployment by way of a capsular structure that can be coupled to an endoscope. FIG. 7A is a schematic illustration of an implantation and deployment capsule 1000 configured for carrying a gastro-duodenal implant in a concentric manner around a portion of an endoscope 1100 according to an embodiment of the disclosure. FIG. 7B is a schematic illustration of an implantation and deployment capsule 1000 configured for carrying a gastro-duodenal implant in an offset manner relative to an endoscope 1100 according to an embodiment of the disclosure. FIG. 7C is a schematic illustration of an implantation and deployment capsule 1000 in an "in-line" configuration with respect to an endoscope 1100. FIG. 7D is a schematic illustration of an implantation and deployment capsule 1000 in an "offset" configuration with respect to an endoscope 1100.

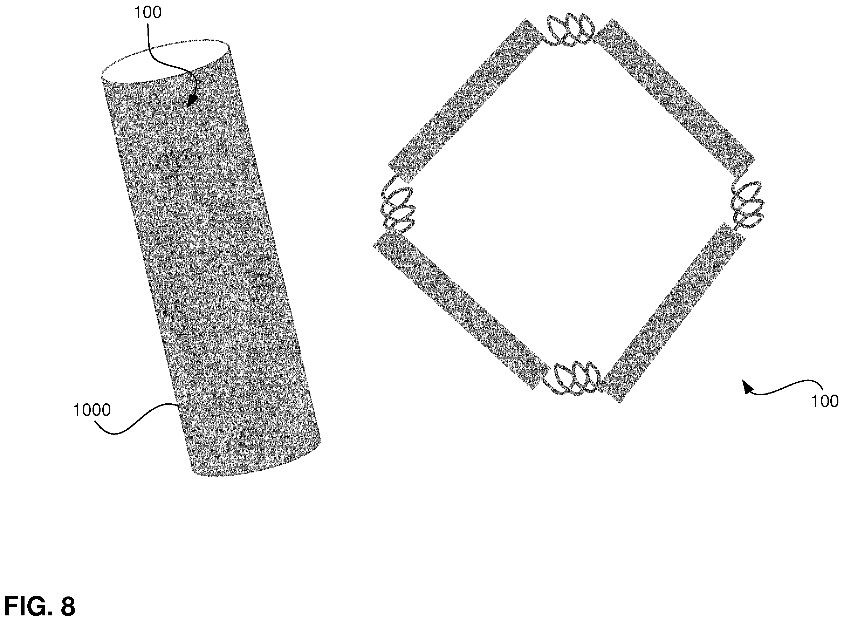

An entire gastro-duodenal implant in accordance with an embodiment of the present disclosure can be carried within an implantation and deployment capsule 1000. FIG. 8 is a schematic illustration showing a first retention structure 100 corresponding to FIG. 2A, which exists in a compressed or minimal profile configuration while disposed within a portion of an implantation and deployment capsule 1000 according to an embodiment of the disclosure. The springs 120 of the first retention structure 100 facilitate the displacement of the first retention structure's frame members 110 relative to each other such that the first retention structure 100 can be disposed within the capsule 1000 in the minimal profile configuration. Upon endoscopic deployment or release of the first retention structure 100 from the capsule 1000, the first retention structure's springs 120 can urge the frame members 110 into the first retention structures default spatial configuration.

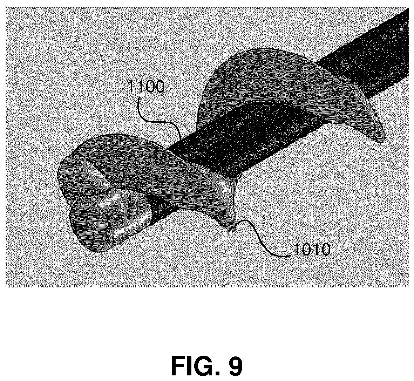

FIG. 9 is a schematic illustration of an implantation and deployment capsule 1010 that includes a serpentine portion surrounding a segment of an endoscope 1100 according to an embodiment of the disclosure. Particular embodiments of a gastro-duodenal device in accordance with the present disclosure can be entirely accommodated within such a serpentine implantation and deployment capsule 1010.

### EXAMPLE 1

An embodiment of a gastro-duodenal implant in accordance with the present disclosure was implanted into a pig. The weight of the implanted pig was charted against that of a control pig of the same gender and starting weight but without an implant. Over the course of 60 days, the weight of the implanted pig increased at a rate significantly less than that of the control pig. 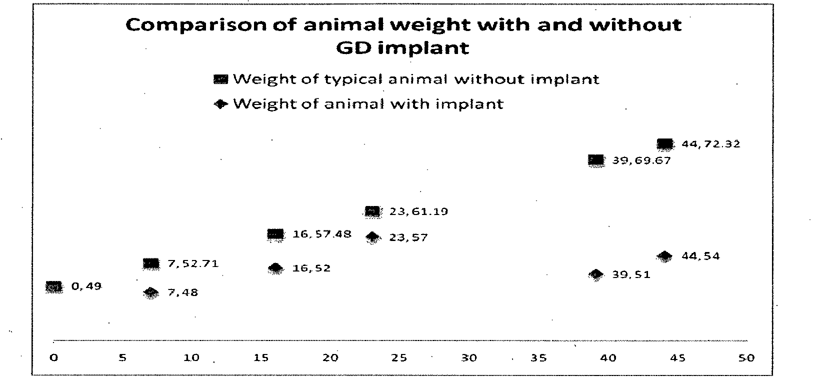 Aspects of particular embodiments of the present disclosure address at least one aspect, problem, limitation, and/or disadvantage associated with exiting gastro-duodenal implants. While features, aspects, and/or advantages associated with certain embodiments have been described in the disclosure, other embodiments may also exhibit such features, aspects, and/or advantages, and not all embodiments need necessarily exhibit such features, aspects, and/or advantages to fall within the scope of the disclosure. It will be appreciated by a person of ordinary skill in the art that several of the above-disclosed systems, components, or alternatives thereof, may be desirably combined into other different systems, components, and/or applications. In addition, various modifications, alterations, and/or improvements may be made to various embodiments that are disclosed by a person of ordinary skill in the art within the scope of the present disclosure.

## Claims

1. A gastro-duodenal implant (10) comprising:
a first retention structure (100) configured for occupying a portion of the stomach;
a second retention structure (200) configured for positioning on the duodenal side of the pyloric opening essentially adjacent to the pyloric sphincter; and
a set of resilient coupling members (300) configured for resiliently coupling the first retention structure (100) and the second retention structure (200),
wherein the set of resilient coupling members (300) facilitates resilient biasing of the first retention structure (100) away from the second retention structure (200); and
wherein the first retention structure (100) is movable within the stomach and contacts with stomach lining following ingestion of food into the stomach and/or during churning motions provided by smooth musculature of the stomach,
**characterized in that**:
the first retention structure (100) comprises a plurality of frame members (110) resiliently coupled together by way of a plurality of springs (120) to form a closed shape,
wherein the plurality of springs (120) facilitates displacement of the plurality of frame members (110) relative to each other towards a deformed spatial configuration of the first retention structure (100) in response to forces exerted upon the first retention structure (100) and biases the plurality of frame members (110) towards a default spatial configuration of the first retention structure (100) when the forces exerted upon the first retention structure (100) are absent,
wherein the closed shape formed by the plurality of frame members (110) and the plurality of springs (120) of the first retention structure (100) has a spatial extent that significantly exceeds cross sectional area of the pyloric opening, in order to minimize likelihood that the first retention structure (100) is accommodated by pyloric sphincter.

2. The gastro-duodenal implant (10) of claim 1, further **characterized in that** the set of resilient coupling members (300) includes a spring (320) configured for displacing portions of the first retention structure (100) relative to the second retention structure (200).

3. The gastro-duodenal implant (10) of claim 1, further **characterized in that** the closed shape of the first retention structure (100) resides within a plane.

4. The gastro-duodenal implant (10) of claim 1, further **characterized in that** the gastro-duodenal implant (10) excludes structural elements that penetrate into or through gastrointestinal lining.

5. The gastro-duodenal implant (10) of claim 1, further **characterized in that** the first retention structure (100) comprises one of (a) a tiered structure, and (b) an enclosure (180) having one of a generally ellipsoidal and a generally toroidal shape, wherein at least two frame members (110) are disposed within the enclosure (180) and are joined together by spring elements (120).

6. The gastro-duodenal implant (10) of claim 1, further **characterized in that** the second retention structure (200) comprises a first ring (210) having a cross-sectional area smaller than the cross-sectional area of the closed shape of the first retention structure (100) and larger than the expected cross-sectional area of a relaxed pyloric opening.

7. The gastro-duodenal implant (10) of claim 6, further **characterized in that** the second retention structure (200) further comprises a second ring (220) coupled to the first ring (210) by way of a flexible sleeve (260).

8. The gastro-duodenal implant (10) of claim 7, further **characterized in that** at least one of the first ring (210) and the second ring (220) comprises an inflatable cuff (210, 250).

9. The gastro-duodenal implant (10) of claim 1, further **characterized in that** the second retention structure (200) comprises one of a tubular member and a trans-pyloric stent.

10. The gastro-duodenal implant (10) of claim 1, further **characterized in that** the gastro-duodenal implant (10) further comprises at least one chute (400) coupled to said second retention structure (200) and configured for positioning along portions of the intestine.

## Patentansprüche

1. Gastroduodenales Implantat (10), umfassend:
eine erste Retentionsstruktur (100), die so konfiguriert ist, dass sie einen Teil des Magens einnimmt;
eine zweite Retentionsstruktur (200), die so konfiguriert ist, dass sie auf der duodenalen Seite des Magenausgangs im Wesentlichen angrenzend an den Pylorus zu liegen kommt; und
eine Menge von elastischen Kopplungselementen (300), die so konfiguriert sind, dass sie die erste Retentionsstruktur (100) und die zweite Retentionsstruktur (200) elastisch aneinander koppeln;
wobei die Menge der elastischen Kopplungselemente (300) die von der zweiten Retentionsstruktur (200) weggerichtete elastische Vorspannung der ersten Retentionsstruktur (100) erleichtert; und
wobei die erste Retentionsstruktur (100) innerhalb des Magens beweglich ist und nach der Aufnahme von Nahrung in den Magen und/oder während Peristaltikbewegungen, die durch die glatte Muskulatur des Magens verursacht werden, mit der Magenschleimhaut in Kontakt kommt, **dadurch gekennzeichnet, dass**:
die erste Retentionsstruktur (100) eine Vielzahl von Rahmenelementen (110) umfasst, die mittels einer Vielzahl von Federn (120) elastisch aneinander gekoppelt sind, wobei eine geschlossene Form entsteht;
wobei die Vielzahl von Federn (120) die Verschiebung der Vielzahl von Rahmenelementen (110) relativ zueinander hinzu einer verformten räumlichen Konfiguration der ersten Retentionsstruktur (100) als Reaktion auf Kräfte, die auf die erste Retentionsstruktur (100) ausgeübt werden, erleichtert und die Vielzahl von Rahmenelementen (110) hin zu einer vorgegebenen räumlichen Konfiguration der ersten Retentionsstruktur (100), wenn die auf die erste Retentionsstruktur (100) ausgeübten Kräfte nicht vorhanden sind, vorspannt;
wobei die von der Vielzahl von Rahmenelementen (110) und der Vielzahl von Federn (120) gebildete geschlossene Form der ersten Retentionsstruktur (100) eine räumliche Ausdehnung hat, die die Querschnittsfläche des Magenausgangs wesentlich überragt, um die Wahrscheinlichkeit, dass die erste Retentionsstruktur (100) durch den Pylorus aufgenommen wird, zu minimieren.

2. Gastroduodenales Implantat (10) gemäß Anspruch 1, weiterhin **dadurch gekennzeichnet, dass** die Menge der elastischen Kopplungselemente (300) eine Feder (320) umfasst, die so konfiguriert ist, dass sie Teile der ersten Retentionsstruktur (100) relativ zu der zweiten Retentionsstruktur (200) verschiebt.

3. Gastroduodenales Implantat (10) gemäß Anspruch 1, weiterhin **dadurch gekennzeichnet, dass** die geschlossene Form der ersten Retentionsstruktur (100) innerhalb einer Ebene liegt.

4. Gastroduodenales Implantat (10) gemäß Anspruch 1, weiterhin **dadurch gekennzeichnet, dass** das gastroduodenale Implantat (10) keine Strukturelemente beinhaltet, die in oder durch die Magen-Darm-Schleimhaut dringen.

5. Gastroduodenales Implantat (10) gemäß Anspruch 1, weiterhin **dadurch gekennzeichnet, dass** die erste Retentionsstruktur (100) entweder (a) eine abgestufte Struktur oder (b) ein Gehäuse (180), das entweder eine im Wesentlichen ellipsoidale oder eine im Wesentlichen toroidale Form aufweist, umfasst, wobei sich wenigstens zwei Rahmenelemente (110) innerhalb des Gehäuses (180) befinden und durch Federelemente (120) miteinander verbunden sind.

6. Gastroduodenales Implantat (10) gemäß Anspruch 1, weiterhin **dadurch gekennzeichnet, dass** die zweite Retentionsstruktur (200) einen ersten Ring (210) umfasst, der eine Querschnittsfläche aufweist, die kleiner ist als die Querschnittsfläche der geschlossenen Form der ersten Retentionsstruktur (100) und größer ist als die erwartete Querschnittsfläche eines entspannten Magenausgangs.

7. Gastroduodenales Implantat (10) gemäß Anspruch 6, weiterhin **dadurch gekennzeichnet, dass** die zweite Retentionsstruktur (200) weiterhin einen zweiten Ring (220) umfasst, der mittels einer flexiblen Hülse (260) an den ersten Ring (210) gekoppelt ist.

8. Gastroduodenales Implantat (10) gemäß Anspruch 7, weiterhin **dadurch gekennzeichnet, dass** der erste Ring (210) und/oder der zweite Ring (220) eine aufblasbare Manschette (210, 250) umfasst.

9. Gastroduodenales Implantat (10) gemäß Anspruch 1, weiterhin **dadurch gekennzeichnet, dass** die zweite Retentionsstruktur (200) ein röhrenförmiges Element oder einen transpylorischen Stent umfasst.

10. Gastroduodenales Implantat (10) gemäß Anspruch 1, weiterhin **dadurch gekennzeichnet, dass** das gastroduodenale Implantat (10) weiterhin wenigstens einen Schacht (400) umfasst, der an die zweite Retentionsstruktur (200) gekoppelt ist und so konfiguriert ist, dass er entlang Teilen des Darms zu liegen kommt.

## Revendications

1. Implant gastro-duodénal (10), comprenant :
une première structure de rétention (100) configurée pour occuper une partie de l'estomac,
une seconde structure de rétention (200) configurée pour se situer sur le côté duodénal de l'ouverture pylorique sensiblement adjacent au sphincter pylorique, et
un ensemble d'éléments de couplage élastiques (300) configurés pour coupler la première structure de rétention (100) élastiquement à la seconde structure de rétention (200),
dans lequel l'ensemble d'éléments de couplage élastiques (300) facilite la sollicitation élastique de la première structure de rétention (100) en éloignement de la seconde structure de rétention (200), et
dans lequel la première structure de rétention (100) est mobile à l'intérieur de l'estomac et entre en contact avec la muqueuse gastrique après ingestion de nourriture dans l'estomac et/ou pendant les mouvements péristaltiques causés par la musculature lisse de l'estomac, **caractérisé en ce que** :
la première structure de rétention (100) comprend une pluralité d'éléments de cadre (110) couplés ensemble élastiquement au moyen d'une pluralité de ressorts (120) pour former une forme fermée,
dans lequel la pluralité de ressorts (120) facilite le déplacement de la pluralité d'éléments de cadre (110) relativement l'un par rapport à l'autre vers une configuration spatiale déformée de la première structure de rétention (100) en réponse à des forces exercées sur la première structure de rétention (100), et sollicite la pluralité d'éléments de cadre (110) vers une configuration spatiale de défaut de la première structure de rétention (100) lorsque les forces exercées sur la première structure de rétention (100) sont absentes,
dans lequel la forme fermée formée par la pluralité d'éléments de cadre (110) et la pluralité de ressorts (120) de la première structure de rétention (100) présente une portée spatiale sensiblement dépassant l'aire de la section transversale de l'ouverture pylorique, afin de minimiser la probabilité que la première structure de rétention (100) est accueillie par le sphincter pylorique.

2. Implant gastro-duodénal (10) selon la revendication 1, **caractérisé en outre en ce que** l'ensemble d'éléments de couplage élastiques (300) comprend un ressort (320) configuré pour déplacer des parties de la première structure de rétention (100) relativement à la seconde structure de rétention (200).

3. Implant gastro-duodénal (10) selon la revendication 1, **caractérisé en outre en ce que** la forme fermée de la première structure de rétention (100) est située dans un seul plan.

4. Implant gastro-duodénal (10) selon la revendication 1, **caractérisé en outre en ce que** l'implant gastro-duodénal (10) ne présente pas d'éléments structurels qui pénétreraient dans ou à travers la muqueuse gastro-intestinale.

5. Implant gastro-duodénal (10) selon la revendication 1, **caractérisé en outre en ce que** la première structure de rétention (100) comprend soit (a) une structure à plusieurs niveaux soit (b) une enceinte (180) ayant soit une forme sensiblement ellipsoïdale soit une forme sensiblement toroïdale, dans lequel au moins deux éléments de cadre (110) se situent dans l'enceinte (180) et sont liés ensemble par des éléments de ressort (120).

6. Implant gastro-duodénal (10) selon la revendication 1, **caractérisé en outre en ce que** la seconde structure de rétention (200) comprend un premier anneau (210) ayant une aire de la section transversale plus petite que l'aire de la section transversale de la forme fermée de la première structure de rétention (100) et plus grande que l'aire de la section transversale attendue d'une ouverture pylorique relaxée.

7. Implant gastro-duodénal (10) selon la revendication 6, **caractérisé en outre en ce que** la seconde structure de rétention (200) comprend en outre un deuxième anneau (220) couplé au premier anneau (210) au moyen d'une manche flexible (260).

8. Implant gastro-duodénal (10) selon la revendication 7, **caractérisé en outre en ce qu'**au moins un parmi le premier anneau (210) et le deuxième anneau (220) comprend une manchette gonflable (210, 250).

9. Implant gastro-duodénal (10) selon la revendication 1, **caractérisé en outre en ce que** la seconde structure de rétention (200) comprend un parmi un élément tubulaire et une endoprothèse transpylorique.

10. Implant gastro-duodénal (10) selon la revendication 1, **caractérisé en outre en ce que** l'implant gastro-duodénal (10) comprend en outre au moins une glissière (400) couplée à ladite seconde structure de rétention (200) et configurée pour se situer le long de parties de l'intestin.
